# EUROPEAN PATENT APPLICATION

(11) **EP 4 740 728 A1**
(43) Date of publication of application: **13.05.2026**
(21) Application number: 25846085.6
(22) Date of filing: 02.09.2025
(51) Int. Cl.: A01K 1/01, A61L 9/12

(54) **DEODORIZATION DEVICE AND PET TOILET**

(30) Priority: 12.09.2024 CN 202422234017 U
(71) Applicant: PETKIT NETWORK TECHNOLOGY (SHANGHAI) CO., LTD., Shanghai 201100 (CN)
(72) Inventor: GUO, Weixue, Shanghai 201100 (CN); HUANG, Jinlong, Shanghai 201100 (CN); ZHANG, Junchao, Shanghai 201100 (CN); MA, Xiaoqing, Shanghai 201100 (CN); JIANG, Lihua, Shanghai 201100 (CN)
(74) Representative: Cabinet Beaumont
(86) International application number: PCT/CN2025/118352
(87) International publication number: WO 2026/056712

(57) **Abstract**

Provided are a deodorizing device and a pet toilet. The deodorizing device includes a housing (1), an aromatherapy box (2), a fan (3), and an image capturing module (4). The housing (1) is disposed on the potty (100). The housing (1) has a first accommodation cavity (11), the aromatherapy box (2) is mounted in the first accommodation cavity (11) and is configured to emit fragrance. The housing (1) also has a second accommodation cavity (12), the fan (3) is mounted in the second accommodation cavity (12), the first accommodation cavity (11) communicates with the second accommodation cavity (12), and the fan (3) is configured to drive air to flow. The housing (1) has an air blowing port (13), the air blowing port (13) is disposed opposite to the potty (100) and communicates with the second accommodation cavity (12). The image capturing module (4) is disposed on the housing (1), is electrically connected to the fan (3), and is configured to photograph a pet in the potty (100).

## Description

The present application claims priority to Chinese Patent Application No. 202422234017.5, filed with the China National Intellectual Property Administration (CNIPA) on Sept. 12, 2024, the disclosure of which is incorporated herein by reference in its entirety.

### TECHNICAL FIELD

The present application relates to the technical field of pet supplies, for example, a deodorizing device and a pet toilet.

### BACKGROUND

Nowadays, with the improvement of the quality of life, an increasing number of people keep a pet at home. Households with pets typically provide a pet toilet, such as a cat litter tray and a dog potty, to satisfy defecation and urination requirements of the pet. However, due to the simple functions and open structure of an existing pet toilet, unpleasant odors are often generated after the pet defecates, especially during hot and humid summers. If users fail to clean the pet toilet promptly, it will bring a poor experience to the users.

### SUMMARY

The present application provides a deodorization device and a pet toilet, which can deodorize and fragrance a potty after a pet defecates, so as to improve the user experience.

An embodiment of the present application provides a deodorizing device. The deodorizing device includes a housing, an aromatherapy box, a fan, and an image capturing module. The housing is disposed on a potty. The housing has a first accommodation cavity, and the aromatherapy box is mounted in the first accommodation cavity and is configured to emit fragrance. The housing also has a second accommodation cavity, the fan is mounted in the second accommodation cavity, the first accommodation cavity communicates with the second accommodation cavity, and the fan is configured to drive air to flow. The housing has an air blowing port, the air blowing port is disposed opposite to the potty and communicates with the second accommodation cavity. The image capturing module is disposed on the housing, is electrically connected to the fan, and is configured to photograph a pet in the potty.

In an embodiment, the housing includes a bottom shell and a mounting shell. The bottom shell is disposed on the potty, the mounting shell is disposed on the bottom shell, the mounting shell is recessed in a first direction to form the first accommodation cavity, and the second accommodation cavity is formed by enclosing the mounting shell and the bottom shell. An abutment block is disposed in the first accommodation cavity, and the aromatherapy box abuts against the abutment block.

In an embodiment, the housing further includes an outer cover, and the outer cover is covered on the aromatherapy box and is detachably connected to the mounting shell.

In an embodiment, the outer cover is provided with vent holes.

In an embodiment, the deodorizing device further includes a sealing inner cover. The sealing inner cover and the image capturing module are disposed in the second accommodation cavity, and the sealing inner cover is covered on the image capturing module and is connected to the housing.

In an embodiment, a side of the housing facing toward the potty is recessed to form a groove, and the image capturing module is disposed in the second accommodation cavity at a position corresponding to the groove.

In an embodiment, a sealant gasket is disposed between the sealing inner cover and the housing.

In an embodiment, the deodorizing device further includes a light supplement module, and the light supplement module is disposed on the housing and is configured to illuminate the pet in the potty.

In an embodiment, an eave plate inclined in a first direction is disposed on the housing, and an end of the eave plate facing away from the housing abuts against the potty vertically.

A pet toilet includes a potty and the above-described deodorizing device is provided.

### BRIEF DESCRIPTION OF THE DRAWINGS

FIG. 1 is an exploded view of a deodorizing device according to a specific implementation of the present application;
FIG. 2 is a longitudinal sectional view of a deodorizing device according to a specific implementation of the present application;
FIG. 3 is a partial enlarged view of A of FIG. 2;
FIG. 4 is a schematic diagram showing an appearance structure of a deodorizing device from a first perspective according to a specific implementation of the present application;
FIG. 5 is a schematic diagram showing an appearance structure of a deodorizing device from a second perspective according to a specific implementation of the present application; and
FIG. 6 is a schematic structural diagram of a pet toilet according to a specific implementation of the present application.

### List of reference numbers

- 100: potty
- 1: housing
- 11: first accommodation cavity
- 111: abutment block
- 12: second accommodation cavity
- 13: air blowing port
- 14: bottom shell
- 141: groove
- 15: mounting shell
- 16: outer cover
- 161: vent hole
- 17: eave plate
- 2: aromatherapy box
- 3: fan
- 4: image capturing module
- 41: camera element
- 42: lens
- 43: pressing plate
- 44: fixing plate
- 45: mainboard
- 5: sealing inner cover
- 6: light supplement module

### DETAILED DESCRIPTION

The present application is described below in conjunction with drawings and embodiments. Embodiments described herein are merely intended to explain the present application and not to limit the present application. For ease of description, only part, not all, of the structures related to the present application are illustrated in the drawings.

In the description of the present application, unless otherwise expressly specified and limited, the term "connected to each other", "connected", or "fixed" is to be construed in a broad sense, for example, as fixedly connected, detachably connected, or integrated; mechanically connected or electrically connected; directly connected to each other, indirectly connected to each other via an intermediary, internally connected between two elements, or an interaction relation between two elements. For those of ordinary skill in the art, the meanings of the preceding terms in the present application may be understood based on situations.

In the present application, unless otherwise expressly specified and limited, when a first feature is described as "above" or "below" a second feature, the first feature and the second feature may be in direct contact or be in contact via another feature between the two features. Moreover, when the first feature is described as "on", "above", or "over" the second feature, the first feature is right on, above, or over the second feature or the first feature is obliquely on, above, or over the second feature, or the first feature is simply at a higher level than the second feature. When the first feature is described as "under", "below", or "underneath" the second feature, the first feature is right under, below, or underneath the second feature, or the first feature is obliquely under, below, or underneath the second feature, or the first feature is simply at a lower level than the second feature.

In the description of this embodiment, the orientation or position relationships indicated by terms "above", "below", "left", "right", and the like are based on the orientation or position relationships shown in the drawings, merely for ease of description and simplifying an operation, these relationships do not indicate or imply that the referred device or element has a specific orientation and is constructed and operated in a specific orientation, and thus it is not to be construed as limiting the present application. In addition, the terms "first" and "second" are used only to distinguish between descriptions and have no special meaning.

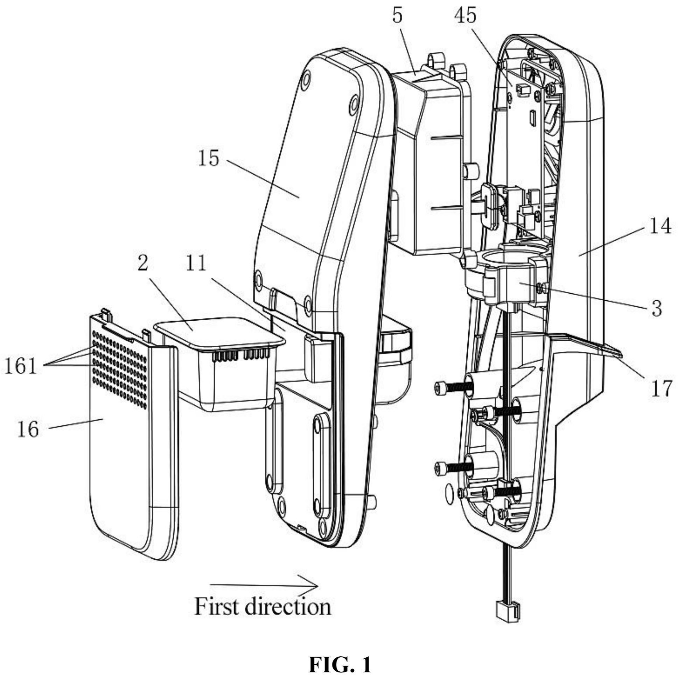

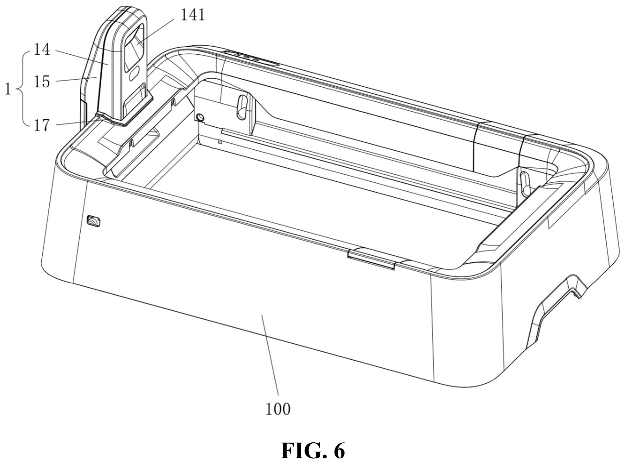

As shown in FIGS. 1, 2, and 6, the present application provides a deodorization device. The deodorization device includes a housing 1, an aromatherapy box 2, a fan 3, and an image capturing module 4. The housing 1 is disposed on a potty 100. The housing 1 has a first accommodation cavity 11, and the aromatherapy box 2 is mounted in the first accommodation cavity 11 and is configured to emit fragrance. The housing 1 also has a second accommodation cavity 12, the fan 3 is mounted in the second accommodation cavity 12, the first accommodation cavity 11 communicates with the second accommodation cavity 12, and the fan 3 is configured to drive air to flow. The housing 1 has an air blowing port 13, the air blowing port 13 is disposed opposite to the potty 100 and communicates with the second accommodation cavity 12. The image capturing module 4 is disposed on the housing 1, is electrically connected to the fan 3, and is configured to photograph a pet in the potty 100. In this embodiment, the aromatherapy box 2 is mounted in the first accommodation cavity 11 of the housing 1, the fan 3 is mounted in the second accommodation cavity 12 of the housing 1, the aromatherapy box 2 is configured to emit fragrance into the air, and the fan 3 is configured to drive the air to flow. The first accommodation cavity 11 communicates with the second accommodation cavity 12, the second accommodation cavity 12 communicates with the air blowing port 13, and the air blowing port 13 is disposed opposite to the potty 100; therefore, the fan 3 can blow the fragrance emitted by the aromatherapy box 2 in the first accommodation cavity 11 to the potty 100 through the air blowing port 13, thereby achieving deodorization and fragrance of the potty 100 and improving the user experience. In addition, the housing 1 is also provided with the image capturing module 4, and the image capturing module 4 is configured to photograph the pet in the potty 100 and is electrically connected to the fan 3; therefore, the fan 3 can receive an electrical signal from the image capturing module 4 and start operation, thereby achieving automatic deodorization of the potty 100 without manual operation by a user, which is time-saving, labor-saving, convenient and efficient. The first accommodation cavity 11 communicates with the second accommodation cavity 12, the second accommodation cavity 12 communicates with the air blowing port 13, the fan 3 is mounted in the second accommodation cavity 12; therefore, under the action of the fan 3, the air flow sequentially passes through the first accommodation cavity 11, the second accommodation cavity 12 and the air blowing port 13, and blows out the fragrance emitted by the aromatherapy box 2 via the air blowing port 13.

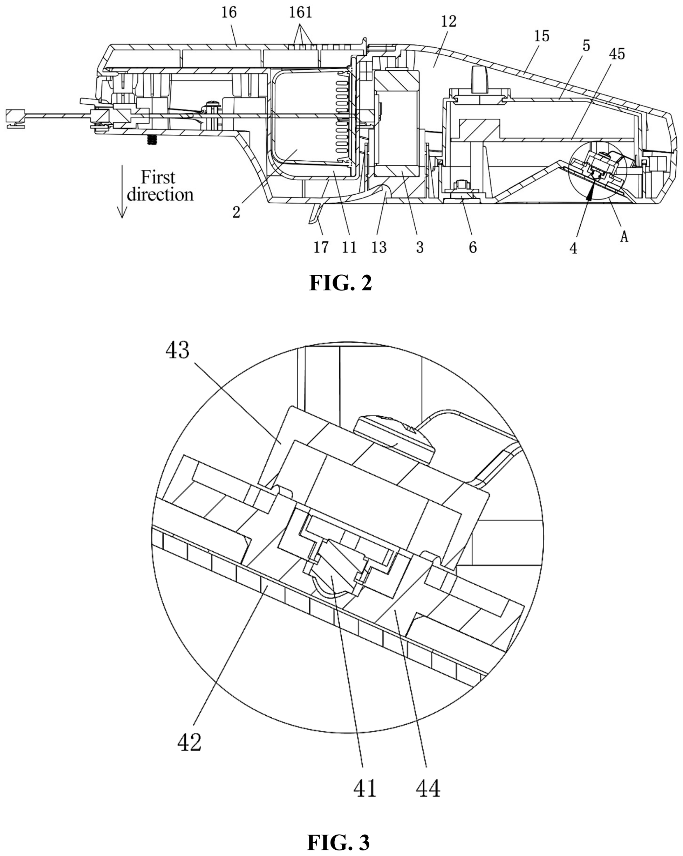

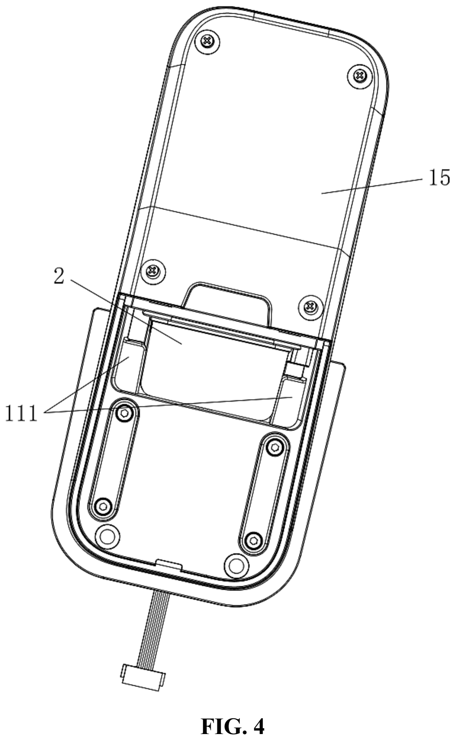

As shown in FIGS. 1 to 4, the housing 1 includes a bottom shell 14 and a mounting shell 15. The bottom shell 14 is disposed on the potty 100, and the mounting shell 15 is disposed on the bottom shell 14. The mounting shell 15 is recessed in a first direction to form the first accommodation cavity 11, and the second accommodation cavity 12 is formed by enclosing the mounting shell 15 and the bottom shell 14. An abutment block 111 is disposed in the first accommodation cavity 11, and the aromatherapy box 2 abuts against the abutment block 111. In this embodiment, an outer cover 16 is hidden in FIG. 3 for the purpose of clearly viewing the internal structure of the first accommodation cavity 11. The bottom shell 14 and the mounting shell 15 are each provided with a through hole, and the through hole on the bottom shell 14 is in one-to-one correspondence with the through hole on the mounting shell 15. A bolt passes through the mounting shell 15 and the bottom shell 14 in sequence and is connected to the potty 100, to lock the mounting shell 15 and the bottom shell 14 and achieve the connection between the bottom shell 14 and the potty 100. The abutment block 111 is disposed in the first accommodation cavity 11, and the abutment block 111 abuts tightly against the aromatherapy box 2; therefore, the aromatherapy box 2 may be fixed in the first accommodation cavity 11 without other fixing structures. The replacement of the aromatherapy box 2 can be achieved by manual plugging and pulling, which is simple in structure and convenient to operate. The first accommodation cavity 11 is formed by a partial recess of the mounting shell 15, and the second accommodation cavity 12 is formed by enclosing the outer bottom surface of the mounting shell 15 and the inner bottom surface of the bottom shell 14.

As shown in FIG. 1, the housing 1 further includes an outer cover 16. The outer cover 16 is covered on the aromatherapy box 2, and the outer cover 16 is detachably connected to the mounting shell 15. In this embodiment, the outer cover 16 is clamped on the mounting shell 15 and is covered on the aromatherapy box 2, to fix the aromatherapy box 2 in the first accommodation cavity 11 and prevent the aromatherapy box 2 from falling out of the first accommodation cavity 11 accidentally. Moreover, the outer cover 16 can also play a protective role for the aromatherapy box 2.

As shown in FIGS. 1 and 2, the outer cover 16 is provided with vent holes 161. In this embodiment, the vent holes 161 are configured to allow air to circulate, so that the air flow can smoothly enter the first accommodation cavity 11 through the vent holes 161 to ensure that the aromatherapy box 2 can normally emit fragrance under the driving action of the fan 3.

As shown in FIGS. 1 and 2, the deodorization device further includes a sealing inner cover 5. Both the sealing inner cover 5 and the image capturing module 4 are disposed in the second accommodation cavity 12. The sealing inner cover 5 is covered on the image capturing module 4, and the sealing inner cover 5 is connected to the housing 1. In this embodiment, the sealing inner cover 5 is covered on the image capturing module 4, to form a protection for the image capturing module 4 located in the sealing inner cover 5 and avoid contamination and damage to the image capturing module 4 caused by external dust and water.

As shown in FIGS. 2, 4, and 5, a side of the housing 1 facing toward the potty 100 is recessed to form a groove 141, and the image capturing module 4 is disposed in the second accommodation cavity 12 at a position corresponding to the groove 141. In this embodiment, the image capturing module 4 includes a camera element 41, a lens 42, a pressing plate 43, a fixing plate 44, and a mainboard 45. The camera element 41 is an image sensor commonly used in the art, the camera element 41 transmits a captured digital image signal to the mainboard 45, and the mainboard 45 is connected to the fan 3 through a connection wire. The mainboard 45 may process and detect the signal transmitted by the camera element 41. When the mainboard 45 detects that the pet finishes defecating or urinating and leaves the potty 100, the mainboard 45 sends an electrical signal to the fan 3 to start the fan 3, and the fan 3 is turned off after running for a preset time period, to complete the removal of unpleasant odors. A first hole slot is formed on the inner wall of the groove 141, the lens 42 is disposed in the first hole slot, and the camera element 41 is disposed in alignment with the lens 42. The groove 141 is recessed on a side of the bottom shell 14 facing toward the potty 100, and the recessed design can prevent the lens 42 from being scratched or collided by the pet or other external foreign matters, thereby forming a protection for the image capturing module 4. The fixing plate 44 is fixed on the inner wall of the bottom shell 14, the fixing plate 44 is provided with a mounting hole configured to mount the camera element 41, and the mounting hole can fix and limit the camera element 41. The pressing plate 43 is connected to the bottom shell 14 through bolts, and the pressing plate 43 abuts against the camera element 41, to firmly fix the camera element 41. In this embodiment, the sealing inner cover 5 is provided with a wiring hole, and the connection wire of the mainboard 45 passes through the wiring hole. Vertical baffles are annularly disposed around the outer circumference of the wiring hole on the sealing inner cover 5, sealant is filled in the baffle, and the connection wire passes through the sealant and is connected to the fan 3, thereby ensuring the sealing performance at the wiring hole.

A sealing gasket is disposed between the sealing inner cover 5 and the housing 1. In this embodiment, the sealing inner cover 5 is connected to the bottom shell 14 through bolts, and the sealing gasket is disposed between the sealing inner cover 5 and the housing 1, to improve the sealing effect of the sealing inner cover 5 and further form an effective protection for the image capturing module 4 located in the sealing inner cover 5.

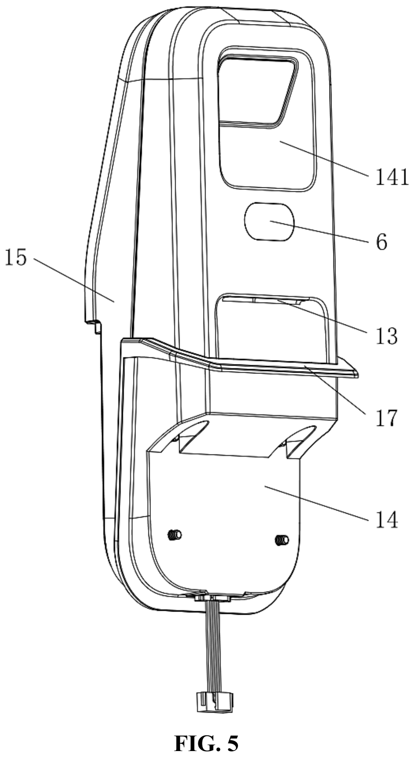

As shown in FIGS. 2 and 5, the deodorization device further includes a light supplement module 6. The light supplement module 6 is disposed on the housing 1 and is configured to illuminate the pet in the potty 100. In this embodiment, the light supplement module 6 includes a light supplement glass sheet and a light source. A second hole slot is formed on the side of the bottom shell 14 facing toward the potty 100, the light supplement glass sheet is disposed in the second hole slot, and the light source is disposed in the second accommodation cavity 12 and is disposed in alignment with the light supplement glass sheet. The light supplement module 6 can provide illumination for the potty 100, so that the images captured by the image capturing module 4 are brighter and clearer.

As shown in FIGS. 2 and 5, an eave plate 17 inclined in a first direction is disposed on the housing 1, and an end of the eave plate 17 facing away from the housing 1 abuts against the potty 100 vertically. In this embodiment, the deodorization device is mounted at the edge of the potty 100, the eave plate 17 is protrudingly disposed on the bottom shell 14, the eave plate 17 is inclined downwards in the first direction, and the end of the eave plate 17 facing away from the bottom shell 14 abuts against the potty 100 vertically, thereby forming a water draining slope structure. This structure can rapidly drain liquid, prevent the liquid from accumulating here and penetrating into the gap between the bottom shell 14 and the potty 100, and further avoid the breeding of bacteria.

The present application further provides a pet toilet. The pet toilet includes a potty 100 and the above-described deodorization device. The potty 100 is configured to collect feces or urine of pets, and the deodorization device can remove unpleasant odors after the pet finishes excretion and leaves the potty 100. The pet toilet has a simple structure and is convenient to use, which can deodorize and fragrance the potty 100. The user can conveniently replace the aromatherapy box 2 according to personal preference, and the whole deodorization process does not need manual operation by the user, which is time-saving, labor-saving, convenient and efficient.

## Claims

1. A deodorizing device, comprising:
a housing (1) disposed on a potty (100);
an aromatherapy box (2), wherein the housing (1) has a first accommodation cavity (11), and the aromatherapy box (2) is mounted in the first accommodation cavity (11) and is configured to emit fragrance;
a fan (3), wherein the housing (1) also has a second accommodation cavity (12), the fan (3) is mounted in the second accommodation cavity (12), the first accommodation cavity (11) communicates with the second accommodation cavity (12), and the fan (3) is configured to drive air to flow; and
an image capturing module (4), wherein the image capturing module (4) is disposed on the housing (1), is electrically connected to the fan (3), and is configured to photograph a pet in the potty (100),
wherein the housing (1) has an air blowing port (13), the air blowing port (13) is disposed opposite to the potty (100) and communicates with the second accommodation cavity (12).

2. The deodorizing device of claim 1, wherein the housing (1) comprises a bottom shell (14) and a mounting shell (15), the bottom shell (14) is disposed on the potty (100), the mounting shell (15) is disposed on the bottom shell (14), the mounting shell (15) is recessed in a first direction to form the first accommodation cavity (11), and the second accommodation cavity (12) is formed by enclosing the mounting shell (15) and the bottom shell (14); and
wherein an abutment block (111) is disposed in the first accommodation cavity (11), and the aromatherapy box (2) abuts against the abutment block (111).

3. The deodorizing device of claim 2, wherein the housing (1) further comprises an outer cover (16), and the outer cover (16) is covered on the aromatherapy box (2) and is detachably connected to the mounting shell (15).

4. The deodorizing device of claim 3, wherein the outer cover (16) is provided with vent holes (161).

5. The deodorizing device of claim 1, further comprising a sealing inner cover (5), wherein the sealing inner cover (5) and the image capturing module (4) are disposed in the second accommodation cavity (12), and the sealing inner cover (5) is covered on the image capturing module (4) and is connected to the housing (1).

6. The deodorizing device of claim 5, wherein a side of the housing (1) facing toward the potty (100) is recessed to form a groove (141), and the image capturing module (4) is disposed in the second accommodation cavity (12) at a position corresponding to the groove (141).

7. The deodorizing device of claim 5, wherein a sealant gasket is disposed between the sealing inner cover (5) and the housing (1).

8. The deodorizing device of claim 1, further comprising a light supplement module (6), wherein the light supplement module (6) is disposed on the housing (1) and is configured to illuminate the pet in the potty (100).

9. The deodorizing device of claim 1, wherein an eave plate (17) inclined in a first direction is disposed on the housing (1), and an end of the eave plate (17) facing away from the housing (1) abuts against the potty (100) vertically.

10. A pet toilet comprising a potty (100) and the deodorizing device of any one of claims 1 to 9.
